# EUROPEAN PATENT SPECIFICATION

(11) **EP 4 054 759 B1**
(45) Date of publication and mention of the grant of the patent: **08.04.2026**
(21) Application number: 20817110.8
(22) Date of filing: 02.11.2020
(51) Int. Cl.: B01J 21/06, B01J 21/08, B01J 35/37, B01J 35/40, B01J 35/61, B01J 35/63, B01J 37/02, B01J 37/08, B01J 37/10, C07D 301/19

(54) **TITANATED CATALYSTS, METHODS OF PREPARING TITANATED CATALYSTS, AND METHODS OF EPOXIDATION**
TITANIERTE KATALYSATOREN, VERFAHREN ZUR HERSTELLUNG TITANIERTER KATALYSATOREN UND VERFAHREN ZUR EPOXIDIERUNG
CATALYSEURS TITANÉS, PROCÉDÉS DE PRÉPARATION DE CATALYSEURS TITANÉS ET PROCÉDÉS D'ÉPOXYDATION

(30) Priority: 04.11.2019 US 201962930268 P
(43) Date of publication of application: 14.09.2022
(73) Proprietor: Lyondell Chemical Technology, L.P., Houston, TX 77056 (US)
(72) Inventor: NAGY, Sandor, Houston, TX 77056 (US); BRUSCHI, Nicholas, Houston, TX 77056 (US); ROSS-MEDGAARDEN, Elizabeth, I., Houston, TX 77056 (US); LEYSHON, David, W., Houston, TX 77056 (US); KIMMICH, Barbara, Houston, TX 77056 (US)
(74) Representative: LyondellBasell
(86) International application number: PCT/US2020/058563
(87) International publication number: WO 2021/091830

(56) References cited:
- WO-A1-01/97967
- WO-A1-2017/080962
- US-A1- 2015 182 959

## Description

### BACKGROUND

Titanated silica systems are catalysts for propylene epoxidation processes that rely on hydroperoxides, such as t-butyl hydroperoxide (TBHP), 1-ethylbutyl hydroperoxide (EBHP), or cumene hydroperoxide (CHP). These processes may include the treatment of silica supports with titanium chloride or one or more titanium alkoxides. Titanium alkoxides and titanium halides, however, may be moisture sensitive, pyrophoric, or a combination thereof. Although relatively inexpensive to purchase, titanium chloride may be moisture sensitive, corrosive, and toxic, thereby making it expensive to handle.

U.S. Patent Application Publication No. 2015/0182959, discloses a process for preparing a titanium catalyst system for epoxidation reactions that includes (i) impregnating a silica carrier with a liquid solution of a titanium compound in an inorganic solvent system, (ii) drying the carrier, (iii) calcinating (i.e., "calcining") the dried product, and (iv) silylating the calcinated (i.e., "calcined") product.

There remains a need for improved processes for preparing the foregoing catalysts, including high-volume commercial catalysts, that are efficient, safer, cheaper, more environmentally friendly, or a combination thereof.

Many fixed bed epoxidation catalysts include titanated silica supports. The supports may have a weight average particle size of 0.2 mm to 3mm, and include unevenly shaped particles. An example of supports is disclosed at WO 2017/080962. The supports of WO 2017/080962 have a surface area of 330 m²/g to 450 m²/g. These supports, however, may suffer from one or more disadvantages, such as a large delta pressure that can be associated with their use in fixed bed reactors.

There remains a need for catalyst supports, including titanated silica supports, that overcome one or more of these disadvantages, and/or perform better in fixed bed reactors. The catalysts of the prior art do not have a sufficient crush strength. The current disclosure provides a solution to this problem by using a spherical catalyst support.

WO 01/97967 discloses a titanated silica catalyst for the epoxidation of propene using hydroperoxides, such as ethylbenzyl hydroperoxide or tert-butyl hydroperoxide. A silica support, hydrolysed with steam and silylated with hexamethyldisilazane.

### BRIEF SUMMARY

Provided herein are methods of preparing titanated silica catalysts az rdported in the claims that are safe, relatively inexpensive, and/or environmentally friendly. The titanated silica catalysts can exhibit improved catalyst performance, such as in epoxidation processes, and the degree of improvement is surprising.

In some embodiments, the methods include providing a silica support that includes a plurality of spherical silica beads having an average diameter of within the range of from 0.1 mm to 5 mm; and contacting the silica support with TiCl₄ vapor to form a titanium-treated silica support. The methods also may include calcinating the titanium-treated silica support to form a calcinated titanium-treated silica support; contacting the calcinated titanium-treated silica support with water vapor; and silylating the calcinated titanium-treated silica support to form a titanated silica catalyst. The plurality of spherical silica beads also may have a surface area of from 400 m²/g to 600 m²/g, a pore volume of from 1 cc/g to 2.5 cc/g, or a combination thereof. Textural properties are measured by nitrogen adsorption isotherms collected at 77 k in the region of P/P0<0.3 (BET surface area) and P/P0>0.95 (pore volume).

In some embodiments, the methods include providing a liquid that includes (i) a water soluble organic compound, and (ii) titanium(IV) bis(ammonium lactato)dihydroxide; and contacting a silica support with the liquid to deposit at least a portion of the titanium(IV) bis(ammonium lactato)dihydroxide on the silica support to form a titanium-treated silica support. The methods may also include calcinating the titanium-treated silica support, and/or
silylating the titanium-treated silica support.

In another aspect, methods of olefin epoxidation are provided. In some embodiments, the methods include providing a titanated silica catalyst described herein or prepared by the methods described herein; and contacting an olefin with the titanated silica catalyst in the presence of an oxidant and in conditions effective to epoxidize the olefin to form an epoxidized olefin.

### DETAILED DESCRIPTION

Provided herein are methods of preparing titanated silica catalysts as reported in the claims. The titanated catalysts provided herein may include a titanium-treated silica support. The titanium-treated silica support may include a plurality of spherical silica beads having (i) an average diameter of from 0.1 mm to 5 mm, (ii) a surface area of from 400 m²/g to 600 m²/g, and (iii) a pore volume of from 1 cc/g to 2.5 cc/g.

### Methods of Preparing Titanated Silica Catalysts

Method of preparing titanated silica catalysts as described in the claims is provided.

spaces of the silica support.

In some embodiments, the impregnating of a silica support includes subjecting the silica support to an incipient wetness impregnation process. In some such embodiments, a vacuum-assisted incipient wetness impregnation process may be used. A vacuum-assisted incipient wetness impregnation process may rely at least in part on capillary action to impregnate a silica support with a liquid. In some embodiments, the methods provided herein include providing a silica support that includes a plurality of spherical silica beads having an average diameter of 0.1 mm to 5 mm; contacting the silica support with TiCl₄ vapor to form a titanium-treated silica support. In some embodiments the plurality of spherical silica beads have an average diameter of 0.75 mm to 2.5 mm. In some embodiments the plurality of spherical silica beads have an average diameter of 1.0 mm to 3.5 mm. In some embodiments the plurality of spherical silica beads have an average diameter of 1.5 mm to 4.25 mm. In some embodiments the plurality of spherical silica beads have an average diameter of 1.75 mm to 2.5 mm. In some embodiments the plurality of spherical silica beads have an average diameter of 0.75 mm to 1.5 mm. The method may also include calcinating the titanium-treated silica support to form a calcinated titanium-treated silica support; contacting the calcinated titanium-treated silica support with water vapor; and silylating the calcinated titanium-treated silica support to form the titanated silica catalyst.

In some embodiments, the methods provided herein include calcinating a titanium-treated silica support; and silylating the titanium-treated silica support to form a titanated silica catalyst.

In some embodiments, the calcinating of the titanium-treated silica support includes subjecting the titanium-treated silica support to an elevated temperature of 100° C to 1,000° C, 300° C to 800° C, or 600° C to 800° C. In some embodiments, the calcinating of a titanium-treated silica support includes heating the titanium-treated silica support in air to a temperature of 500 °C to 750 °C for 1 hour to 3 hours. In some embodiments, a temperature gradient is used. In some embodiments, a titanium-treated silica support is heated to 100° C for 15 minutes, then to 250° C for 15 minutes, and then to 700° C for 2 hours. In some embodiments, the calcination is performed under an inert atmosphere, such as nitrogen or a noble gas. In some embodiments, at least a first portion of the calcination is performed under an inert gas, and then at least a second portion of the calcination is performed in air. In some embodiments, the calcination is carried out in an atmosphere which includes oxygen. In some embodiments, the calcination is carried out in the absence of oxygen.

After the calcinating of a titanium-treated silica support, the calcinated titanium-treated silica support may be washed or steam treated with an alcohol, water, or a combination thereof. The alcohol may include a C₁-C₁₈ hydrocarbyl substituted with at least one hydroxyl moiety.

A washed titanium-treated silica support may be dried. In some embodiments, the drying includes subjecting the washed titanium-treated silica support to an elevated temperature. In some embodiments, the temperature is greater than 50° C. In some embodiments, the temperature is 50° C to 200° C. In some embodiments, the temperature is 100° C to 150° C. In some embodiments, the washed titanium-treated silica support is dried under a stream of an inert gas. In some embodiments, the washed titanium-treated silica support is dried for a time of 0.1 hours to 2 hours. In some embodiments, the washed titanium-treated silica support is dried for a time of 1 hour to 4 hours. In some embodiments, the time is 2 hours.

The silylating of the titanium-treated silica support includes contacting the titanium-treated silica support with a silylating agent. Any silylating agent may be used. In some embodiments, the silylating agent is an organosilane, an organosilylamine, an organosilazane, or a combination thereof. Examples of silylating agents are disclosed at U.S. Patent No. 10,017,484

In some embodiments, silylating agent is an organodisilazane of the following formula:

R₃SiNHSiR'₃,

wherein each R and R' is independently selected from a C₁-C₆ hydrocarbyl. In some embodiments, the silylating agent includes hexamethyldisilazane.

### Silica Support

Any known silica support may be used in the methods provided herein. Non-limiting examples of silica supports include those disclosed at U.S. Patent No. 10,017,484.

The silica support includes a plurality of spherical silica beads. A bead is "spherical" when [1] it is spherical, [2] its smallest diameter is equal to or greater than 95 % of its largest diameter (e.g., a smallest diameter of at least 1.9 mm and a largest diameter of 2 mm), and/or [3] it would satisfy element [1] and/or [2], but for an imperfection, such as a surface imperfection (e.g, trench, depression, etc.). Non-limiting examples of spherical silica beads include AlphaCat^{®} 4000 silica beads available from PQ Corporation (Malvern, Pennsylvania, USA).

In some embodiments, the silica support of the catalyst includes silicon oxide. In some embodiments, the silica support includes silicon oxide and titanium oxide. In some embodiments, the silica support includes at least 90 % by weight of silicon oxide, based on the weight of the silica support. In some embodiments, the silica support includes at least 95 % by weight of silicon oxide, based on the weight of the silica support. The percentage of silicon oxide and one or more other oxides in the silica support may be measured using XRF (x-ray fluorescence spectroscopy). In some embodiments, the one or more other oxides, such as titanium oxide, account for less than 10 % by weight of the silica support, based on the weight of the silica support. In some embodiments, the one or more other oxides account for 0.01 % by weight to 9.9 % by weight of the silica support, based on the weight of the silica support.

In some embodiments, the silicon oxide includes silicon oxide that is flocculated and/or otherwise linked together to form densely packed masses of silica oxide. In some embodiments, the silicon oxide includes synthetic silica powder. The synthetic silica powder may be a powder that is flocculated into open-packed, easily disintegrated, and/or loosely knit aggregates.

In some embodiments, the silica support includes silica-alumina, silica-magnesia, silica-zirconia, silica-alumina-boria, silica-aluminum-magnesia, or a combination thereof. In some embodiments, the silica support includes a plurality of molecular sieves. The plurality of molecular sieves may include large pore and/or mesoporous molecular sieves, such as MCM-41, MCM-48, M41S, or a combination thereof.

The silica support has an average surface area within the range of from 400 m²/g to 600 m²/g. Textural properties are measured by nitrogen adsorption isotherms collected at 77 k in the region of P/P0<0.3 (BET surface area) and P/P0>0.95 (pore volume In some embodiments, the silica support has an average surface area within the range of from 450 m²/g to 550 m²/g. In some embodiments, the silica support has an average surface area within the range of from 400 m²/g to 600 m²/g, and the silica support includes a plurality of spherical silica beads. In some embodiments, the silica support has an average surface area within the range of from 450 m²/g to 550 m²/g, and the silica support includes a plurality of spherical silica beads. In some embodiments, the silica support has an average surface area within the range of from 450 m²/g to 460 m²/g. In some embodiments, the silica support has an average surface area within the range of from 530 m²/g to 540 m²/g.

The silica support has an average pore volume of from 1 g/cm³ to 2.5 g/cm³. In some embodiments, the silica support has an average pore volume of from 1 g/cm³ to 1.5 g/cm³. In some embodiments, the silica support has an average pore volume of from 1 g/cm³ to 2.5 g/cm³, and the silica support includes a plurality of spherical silica beads.

The average pore volume and/or average surface area of a silica support may be measured using nitrogen porosimetry.

In some embodiments, the silica support has an average pore diameter greater than 70 Å. In some embodiments, the average pore diameter of the silica support is 70 Å to 150 Å. In some embodiments, the average pore diameter of the silica support is 90 Å to 110 Å. In some embodiments, the average pore diameter of the silica support is 91 Å to 108 Å.

The silica support may have any desired particle size. In some embodiments, a desired particle size of the silica support is obtained through crushing and/or extruding. In some embodiments, a desired particle size of the silica support is obtained by classifying the silica support through a sieve. In some embodiments, the average diameter of the silica support is less than 5.0 mm. In some embodiments, the average diameter of the silica support is from 0.1mm to 5.0 mm. In some embodiments, the average diameter of the silica support is from 0.2 mm to 4 mm. In some embodiments, the silica support includes a plurality of spherical silica beads having an average diameter of from 0.3 mm to 2 mm. In some embodiments, the silica support includes a plurality of spherical silica beads having an average diameter of from 0.4 mm to 4 mm. In some embodiments, the silica support includes a plurality of spherical silica beads having an average diameter of from 0.5 mm to 2 mm. In some embodiments, the silica support includes a plurality of spherical silica beads having an average diameter of from 0.5 mm to 3 mm. In some embodiments, the silica support includes a plurality of spherical silica beads having an average diameter of from 0.5 mm to 4 mm. In some embodiments, the silica support includes a plurality of spherical silica beads having an average diameter of from 0.75 mm to 3.25 mm. In some embodiments, the silica support includes a plurality of spherical silica beads having an average diameter of from 0.5 mm to 2.5 mm. In some embodiments, the silica support includes a plurality of spherical silica beads having an average diameter of from 1.5 mm to 3.5 mm. In some embodiments, the silica support includes a plurality of spherical silica beads having an average diameter of from 2 mm to 4 mm.

In some embodiments, the silica support is dried before the silica support is contacted with a liquid. In some embodiments, the drying of the silica support includes heating the silica support to a temperature of 100° C to 850° C. In some embodiments, the temperature is greater than 120° C. In some embodiments, the temperature may be in the range of from 150° C to 300° C. In some embodiments, the silica support is dried in a vacuum. In some embodiments, the silica support is dried under a flowing stream of an inter gas, such as nitrogen or a noble gas. In some embodiments, the silica support is dried for a time of from 1 hour to 48 hours. In some embodiments, the silica support is dried for a time of from 2 hours to 24 hours.

A water soluble organic compound may be adsorbed to a silica support. In some embodiments, the silica support includes less than 3 % by weight of carbon, based on the weight of the silica support. In some embodiments, the silica support includes 0.05 % by weight to 3 % by weight of carbon, based on the weight of the silica support. In some embodiments, the silica support includes 1 % by weight to 2 % by weight of carbon from an adsorbed water soluble organic compound and/or other materials. In some embodiments, the carbon content of the silica support is measured using carbon nitrogen analysis by converting the carbon into carbon dioxide at a high temperature.

### Methods of Epoxidation

The catalysts described herein may be used in the production of epoxides from an olefin. Therefore, provided herein are methods of olefin epoxidation. The methods may include contacting an olefin with a titanated silica catalyst, as described herein, in the presence of an oxidant and in conditions effective to epoxidize the olefin to form an epoxidized olefin.

The methods of epoxidation described herein may include batch epoxidation methods, or continuous epoxidation methods.

In some embodiments, the catalysts described herein result in relatively higher conversion of an olefin into a product. In some embodiments, at least 35 mol % of an olefin is converted to an epoxidized olefin in the methods of epoxidation described herein. In some embodiments, at least 45 mol % of an olefin is converted to an epoxidized olefin in the methods of epoxidation described herein. In some embodiments, at least 50 mol % of an olefin is converted to an epoxidized olefin in the methods of epoxidation described herein. In some embodiments, at least 55 mol % of an olefin is converted to an epoxidized olefin in the methods of epoxidation described herein. In some embodiments, at least 65 mol % of an olefin is converted to an epoxidized olefin in the methods of epoxidation described herein. In some embodiments, at least 75 mol % of an olefin is converted to an epoxidized olefin in the methods of epoxidation described herein. In some embodiments, at least 85 mol % of an olefin is converted to an epoxidized olefin in the methods of epoxidation described herein.

Any oxidant, i.e., oxidizing agent, may be used in the methods described herein. In some embodiments, the oxidizing agent is a hydroperoxide. In some embodiments, the hydroperoxide is an alkylhydroperoxide. In some embodiments, the alkyl group has from 1 to 12 carbon atoms. In some embodiments, the alkyl group is tert-butyl. In other embodiments, the hydroperoxide is an aralkylhydroperoxide. In some embodiments, the aralkyl group has from 1 to 24 carbon atoms. In some embodiments, the aralkyl group has from 1 to 12 carbon atoms. In some embodiments, the aralkyl group is ethylbenzyl or cumyl.

In some embodiments, the oxidizing agent is an organic hydroperoxide, such as tert-butyl hydroperoxide (TBHP), cumene hydroperoxide (CHP), ethylbenzene hydroperoxide, or 1-ethylbutyl hydroperoxide (EBHP).

Any olefin may be used in the methods of epoxidation described herein. As used herein, the term "olefin" may refer to any hydrocarbyl, such as a C₁-C₃₀ hydrocarbyl, that includes at least one non-aromatic double bond. In some embodiments, the olefin has 1 to 24 carbon atoms. In some embodiments, the olefin has 1 to 12 carbon atoms. In some embodiments, the olefin is propylene, 1-octene, or a combination thereof. In some embodiments, the olefin is substituted with one or more other functional groups, such as a hydroxyl or halide.

Any ratio of olefin to oxidant may be used in the methods of epoxidation described herein. In some embodiments, the molar ratio of olefin to oxidizing agent is from 1:1 to 20:1, or 10:1 to 12:1.

In some embodiments, at least a portion of an epoxidation reaction occurs in the liquid phase. In some embodiments, the liquid phase includes one or more liquids (e.g., one or more solvents) or inert diluents. In some embodiments, the liquid is a hydrocarbon precursor of the hydroperoxide (e.g., either a corresponding alkane or alcohol). If, for example, the hydroperoxide, in some embodiments, is tert-butyl hydroperoxide, then the liquid that may be optionally used may be tert-butanol.

The methods of epoxidation described herein may be modified by adjusting the pressure and/or the temperature. In some embodiments, the methods of epoxidation are carried out, at least in part, at a temperature within the range of from 25° C to 200° C. In some embodiments, the temperature is within the range of from 50° C to 160° C. In some embodiments, the temperature is within the range of from 70° C to 140° C. In some embodiments, the methods of epoxidation are carried out, at least in part, at a pressure that is from ambient pressure to greater than atmospheric pressure. In some embodiments, the pressure is within the range of from 1.4 to 103 bar (20 psi to 1500 psi). In some embodiments, propylene used as the olefin, and the pressure is within the range of from 28 to 69 bar (400 psi to 1000 psi),

In some embodiments, the epoxidation reaction includes multiple phases. For example, at least a portion of the reactants may be in a gaseous phase, and/or at least a portion of the reactants may be in a liquid phase, and/or at least a portion of the catalyst may be in a solid phase. In some embodiments, both reactants are in the liquid phase, and the catalyst is in the solid phase, such that the catalyst in the reaction mixture is used heterogeneously.

In some embodiments, the methods of epoxidation are performed in any commercially useful reactor. In some embodiments, the reactor is selected from a continuous or batch process reactor. Non-limiting examples of reactors include a fixed bed or a slurry reactor. When any of these reactors are used, the reaction may also include separating the reactants and catalyst from the products. In some embodiments, the methods of epoxidation include a fractional distillation, a selective extraction, filtration, and/or a similar separation technique. In some embodiments, at least a portion of any unreacted reactants, a liquid, and/or a catalyst is reused in the epoxidation reaction.

The terms "a," "an," and "the" are intended to include plural alternatives, e.g., at least one. For instance, the disclosure of "a silica support," "an olefin," is meant to encompass one, or mixtures or combinations of more than one silica support, olefin, unless otherwise specified.

In the descriptions provided herein, the terms "includes," "is," "containing," "having," and "comprises" are used in an open-ended fashion, and thus should be interpreted to mean "including, but not limited to." When methods or systems are claimed or described in terms of "comprising" various components or steps, the methods or systems can also "consist essentially of" or "consist of" the various components or steps, unless stated otherwise.

Various numerical ranges may be disclosed herein. When Applicant discloses or claims a range of any type, Applicant's intent is to disclose or claim individually each possible number that such a range could reasonably encompass, including end points of the range as well as any sub-ranges and combinations of sub-ranges encompassed therein, unless otherwise specified. Moreover, numerical end points of ranges disclosed herein are approximate. As a representative example, Applicant discloses, in one embodiment, that a plurality of spherical silica beads has a pore volume of 1 cc/g to 2.5 cc/g. This range should be interpreted as encompassing values in a range of 1 cc/g to 2.5 cc/g, and further encompasses " " each of 1.1 cc/g, 1.2 cc/g, 1.3 cc/g, 1.4 cc/g, 1.5 cc/g, 1.6 cc/g, 1.7 cc/g, 1.8 cc/g, 1.9 cc/g, 2 cc/g, 2.1 cc/g, 2.2 cc/g, 2.3 cc/g, and 2.4 cc/g, including any ranges and sub-ranges between any of these values.

Throughout this application, the term " " is used to indicate that a value includes a variation of error, such as for the device, the method being employed to determine the value, or the variation that exists among the study subjects. The term " " is used to imply the natural variation of conditions and represent a variation of plus or minus 5% of a value. In some embodiments, the variation is plus or minus 1% of a value.

The processes described herein may be carried out or performed in any order as desired in various implementations. Additionally, in certain implementations, at least a portion of the processes may be carried out in parallel. Furthermore, in certain implementations, less than or more than the processes described may be performed.

Many modifications and other implementations of the disclosure set forth herein will be apparent having the benefit of the teachings presented in the foregoing descriptions and the associated drawings. Therefore, it is to be understood that the disclosure is not to be limited to the specific implementations disclosed and that modifications and other implementations are intended to be included within the scope of the appended claims

### EXAMPLES

The present disclosure is further illustrated by the following examples, which are not to be construed in any way as imposing limitations upon the scope thereof.

### Examples 1 and 2 and Comparative Example (CE) - Titanated Silica Catalysts

In this example, two titanated silica catalysts were prepared and tested in an epoxidation process.

Two silica supports were used in this example to prepare the titanated silica catalysts. The first silica support included AlphaCat^{®} 4000 silica particles (PQ Corporation, USA), which are spherical particles having an average particle diameter of 2 mm to 3 mm, a surface area of 455 m²/g, and a pore volume of 1.06 cc/g. The second silica support included AlphaCat^{®} 4000 silica particles (PQ Corporation, USA), which are spherical particles having an average diameter of 2 mm to 3 mm, a surface area of 533 m²/g, and a pore volume of 1.2 cc/g.

The two silica supports of this example were prepared by treating each silica support with TiCl₄ vapor using a standard titanation procedure (see, e.g., U.S. Patent Application No. 10,017,484). The titanated silica supports were then calcined in air at 700 °C for 2 hours, followed by treatment with water vapor, and a treatment with hexamethyldisilazane (HMDS) at 200 °C.

The performance of each of the titanated silica catalysts prepared with the AlphaCat^{®} 4000 silica particles of this example was tested in an octene/1-ethylbutyl hydroperoxide epoxidation process. The octene epoxidation tests were performed using a POSM oxidizer effluent (~7 - 9 % EBHP in ethylbenzene, EB) washed with caustic and treated with CO₂/H₂O to remove sodium. The testing temperature was 70°C for 3 hours using 0.05 g catalyst in a mixture of 1 mL octene and 5 mL POSM oxidate.

For comparison purposes, the performance of a titanated catalyst support prepared via the foregoing procedure with crushed silica particles also was tested. The results of these tests are depicted at the following table:

**Table 1 - Results of Examples**

| **Example** | **Silica Support** | **Silica Support Shape (Dimensions)** | **Surface Area (m²/g)** | **Pore Volume (cc/g)** | **Conversion (%)** |
|---|---|---|---|---|---|
| CE | Crushed Silica Particles | Non-spherical (1 mm to 2 mm) | 467 | 1.09 | 84.2 |
| 1 | AlphaCat^{®} 4000 Silica Particles | Spherical (2 mm to 3 mm) | 455 | 1.06 | 89.5 |
| 2 | AlphaCat^{®} 4000 Silica Particles | Spherical (2 mm to 3 mm) | 533 | 1.2 | 87.3 |

The data of Table 1 indicate that the spherical silica particles (i.e., silica beads) outperformed the crushed silica particles the epoxidation reaction.

### Propylene Epoxidation Reaction Conditions for Examples

The catalysts were also tested under propylene epoxidation conditions. The reactor ID was 1.57 cm (0.62") ; it had a (0.31 cm (1/8") OD thermocouple well and included an oil jacket for heating. The reactor pressure was 5.5 Mpa (800 psig). The feed to the reactor was 50 g/hr of pure propylene and 150 g/hr of caustic washed and dried EBHP oxidate containing 9% EBHP, 88% ethylbenzene and the remainder was methylbenzyl alcohol and acetophenone. This reactor was heated to convert 50% of the EBHP fed. An axial thermocouple was used to measure the temperature of the catalyst bed. This reactor contained 3 grams of spherical catalyst as described above as Example 1. After 100 hours on stream, the catalyst temperature needed to convert 50% of the EBHP was 54.4 °C (130 °F). After 500 hours on stream, the catalyst temperature needed to convert 50% of the EBHP was 76.7 °C (170 °F). The effluent from this reactor was fed to a second reactor which contained 6 grams of the same Example 1 catalyst having the same hours on stream. The temperature of the second reactor was adjusted to convert 99% of the EBHP fed to the first reactor. At 100 hours on stream, the molar selectivity of propylene oxide produced to EBHP consumed in both reactors was 98.0 %. At 500 hours on stream, the molar selectivity of propylene oxide produced to EBHP consumed in both reactors was 97.1% . After 550 hours on stream, the Example 1 catalyst was removed and its crush strength was measured to be an average of 46.26 N (10.4 lb force).

A similar test was performed on the comparative example (CE) catalyst, described above. The operating conditions were the same as for Example 1 above. This catalyst was non-spherical but was made from crushed silica gel having an approximate diameter of 1 mm. After 100 hours on stream, the first reactor's 3 gram catalyst bed required 60 °C (140 °F) to convert 50% of the EBHP fed to it. After 500 hours on stream, the first reactor's 3 gram catalyst bed required 82.2°C (180 °F) to convert 50% of the EBHP fed to it. The effluent from the first reactor was fed to a second reactor which contained 6 grams of the same catalyst having the same time on stream. The temperature was adjusted to convert 99% of the EBHP. At 100 hours on stream, the molar selectivity of propylene oxide produced to EBHP consumed by both reactors was 96.7%. At 500 hours on stream, the molar selectivity of propylene oxide produced to EBHP consumed by both reactors was 98.0%. After 550 hours on stream, the comparative example (CE) catalyst was removed and its crush strength was measured to be an average of 15.56 N (3.5 lb force).

The conclusion from these examples is that the spherical catalyst of Example 1 is more active and has a much higher crush strength as provided in Table 2 below.

**Table 2 - Results of Examples**

| | Catalyst Temperature Required for 50% EBHP conversion 100 hr TOS | Catalyst Temperature required for 50% EBHP conversion 500 hr TOS | PO selectivity (PO/EBHP)mol At 99% EBHP Conversion 100 hr TOS | PO selectivity (PO/EBHP)mil At 99% EBHP Conversion 500 hr TOS | Catalyst particle Crush Strength After 550 hrs |
|---|---|---|---|---|---|
| Example 1 Spherical 2.5 mm catalyst diameter | 130F | 170 F | 98.0 | 97.1 | 46.26 N (10.4 lb force) |
| Comparative Example 1 mm catalyst diameter | 140 F | 180 F | 96.7 | 97.3 | 15.57 N (3.5 lb force) |
| WHSV | 67 | 67 | 22 | 22 | |

## Claims

1. A method of preparing a titanated silica catalyst, the method comprising:
providing a silica support comprising a plurality of spherical silica beads; contacting the silica support with a titanium compound to form a titanium-treated silica support; calcinating the titanium-treated silica support to form a calcinated titanium-treated silica support;
contacting the calcinated titanium-treated silica support with water, steam or an alcohol to form a water or alcohol calcinated titanium-treated support adduct; and
silylating the water or alcohol calcinated titanium-treated silica support adduct to form the titanated silica catalyst.
wherein the plurality of spherical silica beads has an average surface area within the range of from about 400 m²/g to about 600 m²/g;
wherein the plurality of spherical silica beads has an average pore volume of from about 1 cc/g to about 2.5 cc/g;
wherein the average surface area and an average pore volume are measured by nitrogen adsorption isotherms collected at 77 k in the region of P/P0<0.3 (BET surface area) and P/P0>0.95 (pore volume);
wherein the silica support includes a plurality of spherical silica beads having an average diameter of from 2 mm to 4 mm;
wherein spherical means:
[1] it is spherical,
[2] its smallest diameter is equal to or greater than 95 % of its largest diameter, and/or
[3] it would satisfy element [1] and/or [2], but for an imperfection, such as a surface imperfection.

2. The method of claim 1, wherein the titanium compound is titanium tetrachloride (TiCl₄)

3. The method of claim 1, wherein the alcohol is methanol.

4. The method of claim 1, wherein the plurality of spherical silica beads has an average pore volume of from about 1 cc/g to about 1.5 cc/g.

5. The method of claim 1, wherein the calcinating of the titanium-treated silica support comprises heating the titanium-treated silica support in air to a temperature of about 500 °C to about 750 °C for about 1 hour to about 3 hours.

6. The method of claim 1, wherein the silylating of the calcinated titanium-treated silica support comprises contacting the calcinated titanium-treated silica support with an organodisilazane of the following formula:
R₃SiNHSiR'₃,
wherein each R and R' is independently selected from a monovalent C₁-C₆ hydrocarbyl.

7. The method of claim 1, wherein the silylating agent comprises hexamethyldisilazane.

8. A method of olefin epoxidation, the method comprising:
providing the titanated silica catalyst prepared according to the method of claim 1; and
contacting an olefin with the titanated silica catalyst in the presence of an oxidant and in conditions effective to form an epoxidized olefin.

9. The method of claim 8, wherein the olefin comprises propylene.

## Patentansprüche

1. Verfahren zur Herstellung eines titanierten Siliciumdioxidkatalysators, wobei das Verfahren umfasst:
Bereitstellen eines Siliciumdioxidträgers, der eine Vielzahl sphärischer Siliciumdioxidperlen umfasst;
Kontaktieren des Siliciumdioxidträgers mit einer Titanverbindung, um einen titanbehandelten Siliciumdioxidträger zu bilden;
Calcinieren des titanbehandelten Siliciumdioxidträgers, um einen calcinierten titanbehandelten Siliciumdioxidkatalysator zu bilden;
Kontaktieren des calcinierten titanbehandelten Siliciumdioxidträgers mit Wasser, Wasserdampf oder einem Alkohol, um ein Wasser- oder Alkohol-calciniertes titanbehandeltes Trägeraddukt zu bilden; und
Silylieren des Wasser- oder Alkohol-calcinierten titanbehandelten Siliciumdioxidträgeraddukts, um den titanierten Siliciumdioxidkatalysator zu bilden,
wobei die Vielzahl von sphärischen Siliciumdioxidperlen eine durchschnittliche Oberfläche innerhalb des Bereichs von etwa 400 m²/g bis etwa 600 m²/g aufweist;
wobei die Vielzahl der sphärischen Siliciumdioxidperlen ein durchschnittliches Porenvolumen von etwa 1 cm³/g bis etwa 2,5 cm³/g aufweist;
wobei die durchschnittliche Oberfläche und ein durchschnittliches Porenvolumen durch Stickstoffadsorptionsisothermen gemessen werden, die bei 77K in der Region von P/P0<0,3 (BET-Oberfläche) und P/P0>0,95 (Porenvolumen) aufgenommen werden;
wobei der Siliciumdioxidträger eine Vielzahl von sphärischen Siliciumdioxidperlen mit einem durchschnittlichen Durchmesser von 2 mm bis 4 mm einschließt;
wobei sphärisch bedeutet:
[1] sie ist sphärisch,
[2] ihr kleinster Durchmesser ist gleich oder größer als 95 % ihres größten Durchmessers, und/oder
[3] sie würde Element [1] und/oder [2] erfüllen, jedoch mit einem Mangel, wie einem Oberflächenmangel.

2. Verfahren nach Anspruch 1, wobei die Titanverbindung Titantetrachlorid (TiCl₄) ist.

3. Verfahren nach Anspruch 1, wobei der Alkohol Methanol ist.

4. Verfahren nach Anspruch 1, wobei die Vielzahl der sphärischen Siliciumdioxidperlen ein durchschnittliches Porenvolumen von etwa 1 cm³/g bis etwa 1,5 cm³/g aufweist.

5. Verfahren nach Anspruch 1, wobei das Calcinieren des titanbehandelten Siliciumdioxidträgers Erhitzen des titanbehandelten Siliciumdioxidträgers in Luft auf eine Temperatur von etwa 500 °C bis etwa 750 °C für etwa 1 Stunde bis etwa 3 Stunden umfasst.

6. Verfahren nach Anspruch 1, wobei das Silylieren des calcinierten titanbehandelten Siliciumdioxidträgers Kontaktieren des calcinierten titanbehandelten Siliciumdioxidträgers mit einem Organodisilazan der folgenden Formel umfasst:
R₃SiNHSiR'₃,
wobei jedes R und R' unabhängig ausgewählt ist aus einem einwertigen C₁-C₆-Kohlenwasserstoffrest.

7. Verfahren nach Anspruch 1, wobei das Silylierungsmittel Hexamethyldisilazan umfasst.

8. Verfahren zur Olefinepoxidierung, wobei das Verfahren umfasst:
Bereitstellen des titanierten Siliciumdioxidkatalysators, der nach dem Verfahren gemäß Anspruch 1 hergestellt worden ist; und
Kontaktieren eines Olefins mit dem titanierten Siliciumdioxidkatalysator in Gegenwart eines Oxidationsmittels und unter Bedingungen, die effektiv sind, um ein epoxidiertes Olefin zu bilden.

9. Verfahren nach Anspruch 8, wobei das Olefin Propylen umfasst.

## Revendications

1. Procédé de préparation d'un catalyseur de silice au titane, le procédé comprenant :
la mise à disposition d'un support de silice comprenant une pluralité de billes sphériques de silice ; la mise en contact du support de silice avec un composé de titane afin de former un support de silice traité au titane ;
la calcination du support de silice traité au titane afin de former un support de silice traité au titane calciné ;
la mise en contact du support de silice traité au titane calciné avec de l'eau, de la vapeur ou un alcool afin de former un produit d'addition de support traité au titane calciné et d'eau ou d'alcool ; et
la silylation du produit d'addition de support de silice traité au titane calciné et d'eau ou d'alcool afin de former le catalyseur de silice au titane,
la pluralité de billes sphériques de silice présentant une aire surfacique moyenne dans la plage d'environ 400 m²/g à environ 600 m²/g ;
la pluralité de billes sphériques de silice présentant un volume de pore moyen d'environ 1 cc/g à environ 2,5 cc/g ;
l'air surfacique moyenne et un volume de pore moyen étant mesurés par des isothermes d'adsorption d'azote collectés à 77 K dans la région de P/P0<0,3 (aire surfacique selon BET) et P/P0>0,95 (volume de pore) ;
le support de silice comprenant une pluralité de billes sphériques de silice présentant un diamètre moyen de 2 mm à 4 mm ;
sphérique signifiant que :
[1] l'objet est sphérique,
[2] son plus petit diamètre est égal ou supérieur à 95 % de son plus grand diamètre et/ou
[3] il satisferait à l'élément [1] et/ou [2], mais pour une imperfection, telle qu'une imperfection de surface.

2. Procédé selon la revendication 1, le composé de titane étant du tétrachlorure de titane (TiCl₄).

3. Procédé selon la revendication 1, l'alcool étant le méthanol.

4. Procédé selon la revendication 1, la pluralité de billes sphériques de silice présentant un volume de pore moyen d'environ 1 cc/g à environ 1,5 cc/g.

5. Procédé selon la revendication 1, la calcination du support de silice traité au titane comprenant le chauffage du support de silice traité au titane dans de l'air à une température d'environ 500 °C à environ 750 °C pendant environ 1 heure à environ 3 heures.

6. Procédé selon la revendication 1, la silylation du support de silice traité au titane calciné comprenant la mise en contact du support de silice traité au titane calciné avec un organodisilazane de la formule suivante :
R₃SiNHSiR'₃,
chaque R et R' étant indépendamment choisi parmi un hydrocarbyle en C₁-C₆ monovalent.

7. Procédé selon la revendication 1, l'agent de silylation comprenant de l'hexaméthyldisilazane.

8. Procédé d'époxydation d'oléfines, le procédé comprenant :
la mise à disposition du catalyseur de silice au titane préparé selon le procédé de la revendication 1 ; et
la mise en contact d'une oléfine avec le catalyseur de silice au titane en présence d'un oxydant et dans des conditions efficaces pour former une oléfine époxydée.

9. Procédé selon la revendication 8, l'oléfine comprenant du propylène.
